(19)

Europäisches
Patentamt
European
Patent Office
Office européen
des brevets

(11) **EP 2 161 344 A1**

(12) **EUROPEAN PATENT APPLICATION**

(43) Date of publication:
**10.03.2010 Bulletin 2010/10**

(51) Int Cl.:
***C12Q 1/68*** (2006.01)

(21) Application number: **08020918.2**

(22) Date of filing: **02.12.2008**

<table>
<tr><td>(84) Designated Contracting States:<br><b>AT BE BG CH CY CZ DE DK EE ES FI FR GB GR<br>HR HU IE IS IT LI LT LU LV MC MT NL NO PL PT<br>RO SE SI SK TR</b><br>Designated Extension States:<br><b>AL BA MK RS</b></td><td>(72) Inventor: <b>Holzhauser, Thomas<br>63225 Langen (DE)</b><br><br>(74) Representative: <b>Benedum, Ulrich Max et al<br>Haseltine Lake LLP<br>Theatinerstrasse 3<br>80333 München (DE)</b></td></tr>
<tr><td>(30) Priority: <b>05.09.2008 EP 08015740</b><br><br>(71) Applicant: <b>Paul-Ehrlich-Institut Bundesamt für<br>Sera<br>und Impfstoffe<br>63225 Langen (DE)</b></td><td></td></tr>
</table>

(54) **Method for the quantitative detection of an organic substance in a sample**

(57) The invention pertains to a method for the quantitative detection of an organic substance containing a nucleic acid to be detected in a sample based on a given threshold value for that organic substance in the sample, the use of this method, as well as a kit for practicing this method.

**Figure 1**

```
STEP1:
86 bp PCR product from peanut: The hybridization stretch of the peanut DNA
specific probe is "framed"

1   GCAGCAGTGG GAACTCCAAG GAGACAGAAG ATGCCAGAGC          40
41  CAGCTCGAGA GGGCGAACCT GAGGCCCTGC GAGCAACATC TCATGC   86
    (SEQ ID NO 7)

STEP 2:
Creation of a 21 bp insert for specific binding of the probe for the competitor
dsDNA: reverse sequence of peanut specific probe:

ccggagtccaagcgggagagc (SEQ ID NO 8)

STEP 3:
Creation of a 107 bp competitive dsDNA: Insert the reverse sequence in the mid-
dle of the hybridization stretch of the peanut specific probe to avoid hybridization
of the peanut specific probe

1   GCAGCAGTGG GAACTCCAAG GAGACAGAAG ATGCCAGAGC          40
41  CAGCTCGAGA GGGCGccgga gtccaagcgg gagagcAACC TGAGGCCCTG
    CGAGCAACAT CTCATGC                                 107
    (SEQ ID NO 9)

STEP 4:
Integration of point mutations by inverting neighboring nucleotides (underlined) to
further avoid hybridization with the probe specific for the 86 bp peanut specific
PCR product

1   GCAGCAGTGG GAACTCCAAG GAGACAGAAG ATGCCAGAGC          40
41  CAGCTGCAAG GGCGGccgga gtccaagcgg gagagcACAC TAGGCGCCTG
    CGAGCAACAT CTCATGC                                 107
    (SEQ ID NO 10)
```

EP 2 161 344 A1

**Description**

The invention relates to a method for the quantitative detection of an organic substance in a sample, based on a nucleic acid from that sample, however, without quantifying its nucleic acid. The invention further relates to the use of such a method, and to a kit for the quantitative detection of an organic substance of plant or animal origin in a sample, in particular in a sample of food, feed, stool, or fluids. The invention is particularly useful for detecting and quantifying allergenic foods derived from certain plants or animals in food products without the necessity of external calibration for quantification. Moreover, the invention is useful for the quantification of an organic substance of plant and animal origin in composed matrixes, such as food, feed, body fluid, and stool samples.

There is presently no causative therapy available for individuals that are allergic to certain foods. Therefore, the only effective measure to avoid a potentially life-threatening allergic reaction of an individual to a certain food is to strictly avoid the consumption of the allergenic food itself.

This requires full transparency of food ingredients and food constituents, respectively. Although the labeling of certain allergenic food ingredients is governed by law in many Western and Asian countries, not all known allergenic food ingredients need to be labeled. Moreover, depending on the food regulations, several exemptions from obligatory ingredient labeling are known. In addition, so-called allergen cross-contamination of non-allergenic foods with allergenic foods may occur during production processes of food.

Consequently, to verify legal requirements as well as to control allergen cross-contamination, sensitive, specific, and quantitative methods are needed to screen for the presence of and to determine the amount of allergenic food constituents.

Proteins constitute the allergenic fraction of a food. Currently, quantification of potentially allergenic food ingredients is mainly based on the detection of a specific protein using enzyme-linked immunosorbent assays (ELISA). Usually, polyclonal antisera that are directed against the protein fraction of the potentially allergenic food are applied. Consequently, a major drawback of such ELISAs is that the accuracy of the assay depends on the quality of the antibody or antisera applied and on the protein standard for the quantification of the allergenic food. Since the antibodies and standards for quantification are not standardized, and antibodies may have differing antigen-binding characteristics depending on the choice of immunogen and the immunized animal, the antibody response to specifically detect and quantify allergenic food constituents may be strongly influenced by the choice of antibody and protein standard, respectively. Due to this, ELISA based tests have been demonstrated to provide semi-quantitative results, rather than comparable quantitative results (Poms et al., Food additives and contaminants 2005, 22: 104 - 112).

A further problem related to ELISA methods is that the antiserum used is usually not available in a constant quality over time. That is to say, the characteristics of the antibody might change after a few years, once a different batch of the antibody replaces the initial batch on the market. When this occurs, the ELISA needs to be newly developed and validated, since results may no longer be comparable to the results obtained with the ELISA that was performed with the previously used antibody from a former batch.

It is also known in the state of the art to use nucleic acid-based methods, such as the polymerase chain reaction (PCR) to detect allergenic foods in a sample (e.g. Holzhauser et al., Eur Food Res Technol 2000, 211: 360 - 365). The method described therein is based on the detection of a desired PCR product of a defined size, which is separated using agarose gel electrophoresis. Due to the disadvantages that stem from the reproducibility of the gels, from the mutagenic fluorescent dyes used to detect the DNA, and due to the negative effect of handling PCR amplicons in the laboratories, such as potential cross-contaminations of other PCR samples, the real-time PCR that does not need any post PCR manipulation of the amplicons and that, in addition, does verify the specificity of the amplicon in real-time, has recently been used to detect such food allergens (Hird et al., Eur Food Res Technol 2003, 217: 265 - 268; Stephan and Vieths, J Agric Food Chem 2004, 52: 3754 - 3760). Furthermore, real-time PCR, in contrast to classic PCR with end point detection of the amplicon, is considered to have the potential for quantifying nucleic acids.

Although the DNA to be analyzed in the food sample can in principle be quantified using real-time PCR, a correlation between amplified DNA and the amount of the detected allergenic food constituent cannot be established in most of the cases. This is mainly due to the fact that, depending on the sample matrix, the method for the extraction of nucleic acids, e.g. from food products, yields different amounts of amplifiable DNA.

Moreover, depending on the sample matrix, co-extracted components may have an impact on the efficiency of DNA amplification within each cycle of amplification. Because PCR applies several repetitive cyclic reactions on the same target, the experimental error resulting from the impact of the sample matrix may also be amplified exponentially. This has a large effect on the total PCR efficiency when the extracted nucleic acid is used in a real-time PCR assay.

Consequently, the accuracy of the quantitative results may be extremely poor for an identical amount of amplifiable DNA being present in various specimens.

**Description of the invention**

Accordingly, the problem underlying the present invention was to provide for a means for allowing the quantitative detection of allergenic food components, in particular based on the amplification and detection of nucleic acid stemming from the species causing the allergic reaction, in a sample.

This problem is solved by the present method for the quantitative detection of an organic substance in a sample. In particular, the organic substance can be a potentially allergenic food component derived from an animal species or a plant species. The method is based on a given threshold value for that organic substance in the sample, which represents a certain amount or concentration of the organic substance in the sample. The method makes use of an amplification reaction of a first nucleic acid, the nucleic acid to be detected in the sample that stems from said organic substance.

The method comprises the following steps:

Firstly, a given number of a second nucleic acid molecule is provided as a competitor for an amplification of the nucleic acid to be detected. In an amplification reaction, this second nucleic acid will serve as a second template besides the nucleic acid to be detected. The provided competitor molecule is added to the sample prior to extracting the nucleic acid to be detected from the sample. Thereby, the competitor nucleic acid serves as an internal standard for normalizing the amplification reaction. In an amplification reaction, the given number of the competitor nucleic acid results in a signal that allows for deducing the amount of the organic substance in the sample relative to the given analytical threshold value.

Secondly, a first oligonucleotide molecule and a second oligonucleotide molecule, both for priming an amplification of the nucleic acid to be detected, is provided. The first and the second oligonucleotide should be present in excess over the template molecules, so that the amplification reaction is not impeded due to a lack of oligonucleotides that serve as primers for the amplification reaction. The first and the second oligonucleotide have a nucleotide sequence that allows them to hybridize to the nucleic acid to be detected and to the competitor nucleic acid to prime amplification reactions. It is preferred that the hybridization sites of the first and the second oligonucleotide on the template molecules, i.e. the nucleic acid to be detected and the competitor nucleic acid, are identical for the first oligonucleotide and/or the second oligonucleotide.

Thirdly, the nucleic acid to be detected is extracted from the sample using a suitable extraction method. Since the competitor nucleic acid was previously added to the sample, the competitor nucleic acid is also extracted from the sample with the same or similar efficiency as the nucleic acid to be detected, since the competitor nucleic acid is subject to the same interactions with the sample's matrix material as the nucleic acid to be detected. The amount of competitor nucleic acid necessary for generating an appropriate signal in an amplification reaction needs to be determined prior to the first quantification of the organic substance to be detected. Once determined, the amount of competitor nucleic acid that is added is kept constant in relation to the amount of sample from which the nucleic acid is to be extracted.

Any known extraction method for extracting and purifying nucleic acids from matrix material, such as silica-based nucleic acid extraction or classic CTAB and/or phenol and chloroform extraction, can be applied. Once selected, the applied method for nucleic acid extraction and purification and the down-stream PCR amplification reaction will need to be performed in a reproducible way with the predetermined amount of competitor nucleic acid present throughout nucleic acid extraction to detection. The term matrix material is meant to refer to the specimen in which the nucleic acid to be analyzed is present in the sample.

Fourthly, a first detectable probe for hybridizing with the nucleic acid to be detected is provided, which can generate a first detectable sequence-specific signal. Also, a second detectable probe for hybridizing with the competitor nucleic acid is provided to generate a second detectable sequence-specific signal. By hybridizing with the amplification products, the probes emit a detectable signal. In order to differentiate which signal stems from the amplification of which nucleic acid molecule, the first detectable probe is labeled differently than the second detectable probe. The relative difference between the position of the specific signal of the nucleic acid to be detected and of the competitor nucleic acid is a means to quantify the organic substance in a ratio to the analytical threshold set up initially.

When the amplification is performed using real time polymerase chain reaction, the amplification of nucleic acids results in a certain position of a sequence-specific signal of amplification of the nucleic acid to be detected in a graph showing signal over time (or number of amplification cycles). The position of sequence specific amplification for both types of amplifiable nucleic acid, target and competitor, is preferably determined at an identical and predefined magnitude of signal. The position of sequence specific amplification at this predefined constant magnitude of signal is called "threshold cycle".

Fifthly, an amplification reaction is performed, in which both the nucleic acid to be detected and the competitor nucleic acid are simultaneously amplified, using the same oligonucleotides as primers. The amplification is preferably an enzymatic amplification. In case the enzyme based amplification reaction is a polymerase based amplification reaction, in particular a polymerase chain reaction (PCR), it is preferred that the enzyme is a heat-stable polymerase. However, it is also possible to apply other enzymatic amplification reactions know to a person of skill in the art, including ligase-mediated amplifications (e.g. ligase-chain reaction) or amplifications based on transcription (e.g. NASBA®, 3SR®, TMA®). As an enzyme, a polymerase, such a Taq or Pfu or a ligase can be used, as appropriate for the amplification reaction performed.

Sixthly, the signal stemming from a first detectable probe and the signal stemming form the second detectable probe are detected for determining the quantitative amount of the organic substance under investigation, based on its nucleic acid to be detected. Therefrom, the amount of the organic substance in the sample relative to the given threshold value is deduced. Preferably, in case a real time PCR is used, the position of the signal originating from the nucleic acid to be detected in relation to the position of the signal originating from the competitor nucleic acid is determined.

In short, the invention provides for a means for quantifying an organic substance without quantifying its nucleic acid, e.g. from a food component, by providing an internal standard that is carried along during both the extraction and the analysis phase of the sample. When the method is performed using real time PCR, the difference of the position (threshold cycle) of a sequence specific signal originating from a nucleic acid to be detected in relation to a competitor nucleic acid is detected by providing an internal standard that is carried along during both the extraction and the analysis phase of the sample. The internal standard has an amplification efficiency that is identical or nearly identical to the amplification efficiency of the nucleic acid to be detected. The term "amplification efficiency" refers to the binding efficiency of the nucleotides used to prime an amplification reaction and the efficiency of the amplification reaction itself, e.g. the factor that the number of molecules of a particular template species is amplified by in each round of the amplification reaction.

With the method of the invention, the disadvantage of potentially non-reproducible antibody preparations and protein standards is encountered by the use of a DNA-based methodology, such as polymerase chain reaction (PCR), of which the complete chemistry including recombinant polymerase enzyme is of a reproducible and therefore consistent quality.

The nucleic acid is preferably DNA, but can also be RNA, such as mRNA that is subject to a reverse transcription reaction prior to the amplification reaction.

Ideally, the template nucleic acid, be it the nucleic acid to be detected or the competitor nucleic acid, does not exceed 200 base pairs (bp) in length. Preferred is a length of between 50 bp to 180 bp. The first and/or second oligonucleotide for priming the reaction should have a length of between 12 bp to 30 bp in length. The first and /or second detectable probe should not exceed 40 bp in length.

In a preferred embodiment of the method according to the invention, the nucleic acid to be detected and the competitor nucleic acid both comprise a first sequence portion for hybridisation with the first oligonucleotide and a second sequence portion for hybridisation with the second oligonucleotide. The first detectable probe and the second detectable probe hybridize preferably between the first and the second sequence portion for hybridisation with the oligonucleotides.

The term "hybridisation" refers to the formation of a double stranded nucleic acid molecule consisting of a template nucleic acid and an oligonucleotide or a probe under both stringent and non-stringent conditions, whatever is suited for the analysis method.

It is preferred that the first sequence portion of the nucleic acid to be detected and/or of the competitive nucleic acid contains a sequence that is reverse complementary to the sequence of the first oligonucleotide and/or that the second sequence portion of the nucleic acid to be detected and/or of the competitive nucleic acid contains a sequence that is reverse complementary to the sequence of the second oligonucleotide. In the most preferred embodiment, the oligonucleotides serving as primers for the amplification reaction hybridize perfectly, e.g. without mismatch, with their respective template molecules.

In other words, it is preferred that the first sequence portion of the nucleic acid to be detected and the first sequence portion of the competitor nucleic acid are identical, and/or that the second sequence portion of the nucleic acid to be detected and the second sequence portion of the competitor nucleic acid are also identical.

Besides using the same set of oligonucleotides as primers for both template molecules (e.g. the nucleic acid to be detected and the competitor nucleic acid), it is also preferred that the amplification product of the nucleic acid to be detected and of the competitor nucleic acid are essentially identical in length and of a similar composition of nucleic acid bases, which facilitates equal amplification efficiency in the amplification of the two template species. The term amplification product refers to the product of the amplification reaction obtained by using the first oligonucleotide and the second oligonucleotide. The length of the two amplification products differs preferably less than 15 %, most preferably less than 5 %. This length difference between the amplification product of the nucleic acid to be analyzed and the competitor nucleic acid is preferably located in the sequence portion of the amplification products to which the respective probe hybridizes to, and/or to the portion that at least one of the oligonucleotides hybridizes with. Nonetheless, differences in length between target nucleic acid and competitor nucleic acid may be acceptable if both nucleic acids result in amplification of comparable efficiencies.

It is further preferred that the amplification product of the nucleic acid to be detected and the amplification product of the competitive nucleic acid differ in sequence in 1 to 20 nucleotides (nt), or up to 15%.

It is most preferred that the nucleic acid to be detected and the competitor nucleic acid differ in their sequence only at a sequence portion where the detectable probe hybridizes. Therefore, the first detectable probe and the second detectable probe differ in one embodiment only in one position in the sequences, since this is sufficient under stringent amplification conditions to differentiate between the amplification products. This further contributes to identical amplification efficiency for both template molecules. In another embodiment, the first detectable probe and the second detectable probe differ in between 30 and 2 positions in their sequences.

The step of determining the quantitative amount is preferably performed by comparing the position of the signal stemming from the first probe with the position of the signal stemming from the second probe.

The amount of competitor nucleic acid molecules can either be known as absolute copy numbers or can be determined empirically through a serial dilution of a competitor stock solution of unknown concentration. In a preferred embodiment a calibration is done as follows: The competitor nucleic acid is added to a constant amount of sample matrix that contains the organic substance at a definite amount, such as an analytical threshold, is coextracted with the nucleic acid to be detected, amplified and detected in a way that both signals, of the nucleic acid to be detected and of the competitor nucleic acid, appear at the same position (threshold cycle) in a real time PCR. According to this procedure, the competitive real-time PCR with sequence specific primers and fluorescence probes is calibrated to a desired amount of the species to be detected and quantified without necessarily knowing the quantitative number of amplifiable copies of the nucleic acid to be detected. This way, the calibration can be done for a given amount of species to be detected in a sample matrix, such as a threshold provided by law for the presence of an allergenic food ingredient to be labelled.

As an example, the calibration with competitor can be based on a food sample that contains an allergenic ingredient, such as whole peanut, in an amount that makes labelling the ingredient legally obligatory. The amount of competitor nucleic acid necessary needs to be determined empirically, as mentioned above. The determined amount of competitor nucleic acid to achieve essentially identical or similar signal positions for both the nucleic acid to be detected and the competitor nucleic acid is added to a given amount of sample that contains an unknown amount of the ingredient to be detected and quantified. In other words, first an appropriate amount of competitor nucleic acid needs to be determined that yields an appropriate signal in a suitable amplification reaction. Then, this appropriate amount is added to the sample of unknown amount of organic substance as an internal standard. The amount of competitor nucleic acid is chosen such as to represent the threshold value of the organic substance in the sample.

If both signals, from the nucleic acid of the ingredient to be detected and of the competitor nucleic acid, appear at the same threshold cycle, it can be concluded that the analyzed sample matrix contains the ingredient species to be detected in an amount equal to the level the method was calibrated for using the competitor nucleic acid. If the competitor signal appears later in a real time PCR, i.e. at a higher $C_T$ value, than the signal of the nucleic acid to be detected (the target species), it can be concluded that the sample matrix contains more of the target species than the amount the method was calibrated for with the competitor nucleic acid. Vice versa, if the competitor signal appears earlier in a real time PCR, i.e. at a lower $C_T$ value, than the signal of the sample matrix, the sample matrix does contain less of the target species than the calibration level. Thus, absolute quantification is achieved at the level of calibration.

Moreover, a semi-quantitative conclusion can be drawn if the positions of the signal from the competitor nucleic acid and of the nucleic acid to be detected differ from each other. Specifically, it can be concluded that the amount of nucleic acid to be detected in the sample is "higher" or "lower" than the amount that the threshold value stands for.

If the efficiency of both the amplification, e.g. the real time PCR, and the hybridization reactions of the first and the second detectable probes are identical and around 100 %, the absolute difference in threshold cycles between both targets can be used to calculate the amount of species in the sample as a factor in comparison to the calibrated level and without further need for efficiency control. Then, a difference of 3.332 cycles between the real time PCR signals forms the basis of a factor that represents the difference in the amount of the nucleic acid to be detected relative to the calibrated threshold value. Specifically, a difference of 3.332 amplification cycles between the signal from the nucleic acid to be detected (e.g. peanut) and from the competitor nucleic acid (with the position of the signal from the nucleic acid to be detected being lower) would allow the conclusion that the amount of peanut is 10 times higher in the sample than the amount that is represented by the calibrated analytical threshold value.

It is furthermore possible to use an external standard for the nucleic acid to be detected and/or for the competitor nucleic acid, to calculate the ratio between the amplicons of the nucleic acid to be detected and of the competitor nucleic acid, and to relate this ratio to the calibrated threshold of organic substance.

The method according to the invention is therefore preferably an absolute quantitative detection method.

It is preferred that first detectable probe and the second detectable probe are each tagged with a detectable label. Such a label can be for example FAM, HEX, Cy3 or Cy5. In order to distinguish between the probes, the detectable label of the first detectable probe is distinguishable from the detectable label of the second detectable probe.

It is preferred that the detectable label is suitable for a real-time assay. The probe can be modified, for example with a quencher and/or a label for detection as known by a person of skill in the art, like the dye FAM or the quencher BHQ black hole or dabcyl.

The problem underlying the present invention is also solved by the use of a method described above and herein for the quantitative detection of an organic substance in a sample, based on its nucleic acid, in particular for the quantitative detection of food allergens, bacteria, viruses, mutations (like SNPs) in a sample. The sample can be a food sample, a tissue, an organ, and a body fluid. The term body fluid is meant to comprise fluids such as whole blood, blood plasma, blood serum, urine, sputum, tears, sweat, saliva, lymph fluid, liquor or alike. In such a use, a sample containing a nucleic acid provided.

The problem underlying the present invention is furthermore solved by a kit for the quantitative detection of an organic substance in a sample. Such a kit comprises

a) a given number of a second nucleic acid molecule as a competitor for the nucleic acid to be detected, i.e. as a second template or as an internal standard,
b) a first oligonucleotide and a second oligonucleotide for priming an amplification of the nucleic acid to be detected, wherein the first and the second nucleotide hybridize to the nucleic acid to be detected and to the competitor nucleic acid to prime an amplification reaction, and
c) a first detectable probe for hybridising with the nucleic acid to be detected to generate a first signal from which the position of the signal at defined signal intensity can be determined, and a second detectable probe for hybridizing with the competitor nucleic acid to generate a second signal from which the position of the signal at defined signal intensity can be determined. It is preferred that the first detectable probe and the second detectable probe carry dif ferent labels to make them distinguishable.

The kit can further comprise an enzyme for the amplification of the nucleic acid to be detected and for the amplification of the competitor nucleic acid. Such an enzyme is preferably a polymerase, in particular a heat-stable polymerase such as Taq or Pfu. The kit can further comprise a means for detecting the signal from the first detectable probe and/or from the second detectable probe.

The invention also relates to the use of a kit as described above and herein for performing a method as described above and herein.

The invented method is based on a competitive Polymerase Chain Reaction setting to quantify an organic substance. Since the underlying example is performed in a real-time PCR cycler environment, this invention is called a calibrated real-time cqPCR.

**Figures**

**Figure 1:** Generation of a 107 bp competitive nucleic acid in the form of a competitive double strand DNA with conserved primer binding sites but a divergent binding site for detectable probes for discriminating between the 86 bp long peanut specific PCR product and the competitive nucleic acid. The overall nucleotide composition is nearly conserved between the two amplifiable sequences. The melting temperatures differ only in 2 K. All DNA sequences are displayed from left to right from the 5' to the 3' end.

**Figure 2:** Purified 107 bp competitive DNA at different amounts (lanes 1-3) in comparison to 100 bp DNA ladder (lane M) in agarose gel electrophoresis.

**Figure 3:** Detection of the PCR products of a competitive PCR in agarose gel electrophoresis (M, size marker; N, no template control). Copy numbers of amplified 107 bp competitor nucleic acid and 86 bp nucleic acid to be detected in the form of peanut DNA are shown as orders of approximate magnitude.

**Figure 4:** Comparable efficiencies of nearby 100 % for both the amplification of the peanut DNA (nucleic acid to be detected, killed squares) and the competitor DNA (competitor nucleic acid, filled circles).

**Figure 5:** Primary titration of competitor copies versus chocolate as that contains peanut (nucleic acid to be detected) at the 100 ppm level (75.9 ppm).

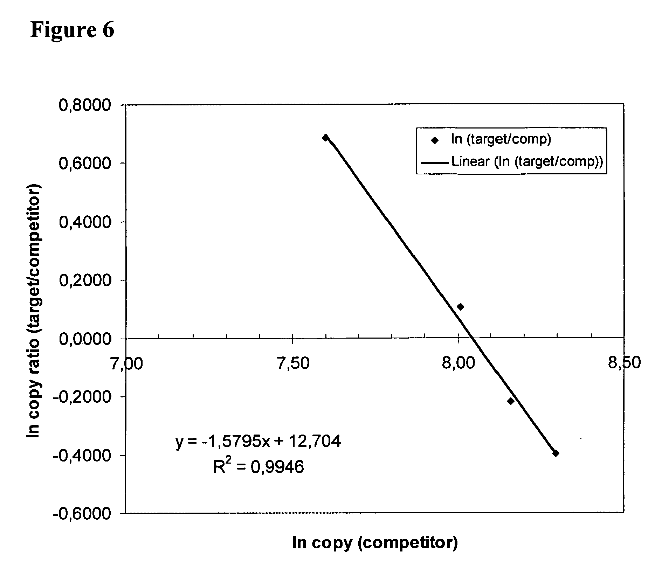

**Figure 6:** Final titration of competitor copies versus chocolate that contains peanut (nucleic acid to be detected) at the 100 ppm level (75.9 ppm).

**Examples**

*Methods*

*Extraction of nucleic acid in the form of DNA from food*

A representative sample was ground to homogeneity with an analytical mill (IKA M20, IKA Labortechnik, Staufen, Germany). Of the homogenized sample, exactly 300 mg (+/- 15 mg) were transferred to a 2 ml microreaction tube and suspended in 1.5 ml of CTAB buffer (55mmol/l hexadecyltrimethyl-ammoniumbromide (CTAB), 1400 mmol/l NaCl, 20 mmol/l ethylenediamintetraacetate (EDTA), 100 mmol/l TRIS (tris(hydroxymethyl)-aminomethane), pH 8.0, adjusted with 10 % HCl, autoclaved). Extraction was carried out in a thermomixer (no. 5437, Eppendorf, Hamburg) at 65 °C, 1000 rpm, for 5 min or until a homogeneous suspension was achieved. After addition of 20 µl of 20 mg/ml Proteinase K (Merck, no. 1.24853, DNA grade, Darmstadt, Germany), the sample was digested at 65 °C, 1000 rpm, for 30 min. For removal of undissolved material, the tube was centrifuged at 14,000 rpm for 2 min in a common desk top centrifuge for microtubes. Subsequently, digested proteins and Proteinase K were removed by chloroform extraction. Therefore, 800 µl of the supernatant were transferred to a 2 ml microreaction tube and 600 µl of chloroform were added. Peptides were precipitated by strong agitation on a vortexer. The tube was centrifuged as described above and 600 µl of the DNA-containing upper phase were transferred to another 1.5 ml microreaction tube. After addition of 1 µl of 20 mg/ml glycogen (Roche, no. 10901393001, for molecular biology) and 500 µl of 80 % isopropanol, the DNA was precipitated by strong agitation on a vortexer for 30 sec. The precipitate was centrifuged as described above and the supernatant was discarded. The resulting DNA pellet was washed once by addition of 500 µl of 70 % ethanol and the ethanol was removed. The pellet was dried at 50 °C for 5 min in the thermomixer. The dried pellet was dissolved at 4°C overnight in 100 µl of TE buffer (10 mmol/l TRIS, 1 mmmol/l EDTA, pH 8.5, adjusted with 10 % HCl, autoclaved) or as much as need to fully dissolve the pellet.

One-hundred microliter of the extracted DNA was further purified by use of the QIAquick PCR Purification Kit (Qiagen, Hilden, no. 28106) according to the protocol using a microcentrifuge. Briefly, 500 µl of buffer PB were added to 100 µl of extracted DNA and the protocol was strictly followed according to the manufacturer's instructions. The purified DNA was eluted by 50 µl of buffer EB from the column. The column was once more eluted with 50 ml of the first elution.

The purified DNA was subjected to PCR analysis or stored at -20 °C until use.

*Normalization of nucleic acid extraction by the addition of competitor nucleic acid*

The DNA extraction was performed identical as described above except for the addition of competitor nucleic acid: Prior to the addition of 20 µl of 20 mg/ml Proteinase K (Merck, no. 1.24853, DNA grade, Darmstadt, Germany), the titrated amount of competitor nucleic acid was pipetted in at total volume of 7.5 µl of 10 mmol/l Tris-Cl, pH 8.5. After the addition of proteinase K the protocol was again strictly followed as described above.

*Fist and second oligonucleotides, first and second detectable probes*

The peanut specific primers and probes for genomic peanut DNA and nucleic acid competitor as well as the synthetic oligonucleotides for the generation of competitor dsDNA were commercially synthesized by commercial suppliers (Biomers, Ulm, Germany; and Biotez, Berlin, Germany). All oligonucleotides are displayed from left to right from the 5' to the 3' end. The hybridizing ends of the competitor synthesis oligonucleotides are underlined.

Forward primer: gcagcagtgggaactccaaggagaca (SEQ ID NO 1)
Reverse primer: gcatgagatgttgctcgcag (SEQ ID NO 2)
Peanut DNA specific probe: FAM-cGAGAGGGCGAACCTGAGGCC-BHQ (SEQ ID NO 3)
Competitor DNA specific probe: HEX-CCGGAGTCCAAGCGGGAGAGC-BHQ (SEQ ID NO 4)
Synthesis oligo #1:

GCAGCAGTGGGAACTCCAAGGAGACAGAAGATGCCAGAGCCAGCTGCAAGGGCG

<u>GCCGGAGTCCAAGCGGGAGAGC</u>  (SEQ ID NO 5)

Synthesis oligo #2:
GCATGAGATGTTGCTCGCAGGCGCCTAGTGT<u>GCTCTCCCGCTTGGACTCCGG</u> (SEQ ID NO 6)

*Generation of competitor nucleic acid*

First, each 50 pmol of the synthesis oligos #1 and #2 were mixed in 20 $\mu$l of 1X PCR buffer (without MgCl$_2$), heated at 96 °C for 30 sec, and cooled to ambient temperature for hybridization. Second, 2 $\mu$l of the hybridization mixture were added to a total volume of 20 $\mu$l of 1X Klenow buffer (10 mmol/l Tris-CL, 5 mmol/l MgCl$_2$, 3.75 mmol/l DDT, pH 7.5) that contains 4 Units of Klenow Fragment (Roche Applied Science, # 11008404001) and 0.1 mmol/l of each dNTP (dATP, dTTP, dGTP, and dCTP). After incubation at 37 °C for 30 min, the 20 $\mu$l of Klenow reaction mixture was purified with a Centri Spin 20 column (Princeton separation, # CS-200, via EMP Biotech GmbH, Berlin) according to the manufacturer's instructions. Then, 5 $\mu$l of purified competitor nucleic acid were added to a total volume of 50 $\mu$l PCR reaction mix that contains 1X PCR buffer A, 800 $\mu$M dNTP mix (each 200 $\mu$M dATP, dTTP, dGTP, and dCTP), 2 mmol/l MgCl$_2$, and 1.25 Units of Platinum® *Taq* (Invitrogen, Karlsruhe, Germany). PCR was performed with 40 cycles of 30 sec at 95 °C, 30 sec at 60 °C and 30 °C at 72 °C. The 107 bp competitor PCR product was purified with a Centri Spin 20 column. The quality of the purified competitor DNA was investigated by agarose gel electrophoresis with subsequent ethidium bromide fluorescence staining: 5 $\mu$l of PCR product and 1 $\mu$l of 6X gel loading concentrate (15 % Ficoll, 0.25 % bromophenol blue, 0.25 % xylenecyanol in destilled water) were mixed, and PCR products separated on 3 % agarose (Carl Roth, no. K 297.2, Karlsruhe, Germany) gels by rapid agarose gel electrophoresis (RAGE) (Cascade Biologics, via TEBU, Frankfurt/M, Germany) according to the manufacturer's instructions. Thereafter, gels were soaked in 0.5 $\mu$g/ml of ethidium bromide for 20 min. Thereafter, PCR products were visualized on a UV transilluminator (TFM20, UVP, via Merck). The size of the PCR products was controlled by comparison with a 100 bp size marker (GenSura, SLL 101,visa Carl Roth) and documentation of results was done with an instant camera (Polaroid GelCam, film type 667, 1/4 sec, 5.6f).

For determination of the concentration of genomic DNA, 5 $\mu$l of DNA was measured at 260 nm wavelength in a microcuvette in a spectrophotometer. One OD of dsDNA at 260 nm was equivalent to 50 ng/$\mu$l of genomic DNA. From the determined amount of competitor DNA, the copy number was calculated considering the nucleic acid composition and length of the competitor dsDNA.

The competitor DNA PCR product was serially diluted in TE buffer (10 mmol/l TRIS, 1 mmol/l EDTA, pH 8.5) containing 0.1 % Tween 20, and stored at -20 °C until use.

*Generation of the purified peanut DNA amplicon*

Genomic DNA was purified from peanut as described above. As described above for the competitor DNA, the PCR was performed with peanut genomic DNA to generate an 86 bp peanut PCR product. The peanut PCR product was further purified, quantified by UV spectrophotometer, serially diluted, and stored as described above.

*Real-time cqPCR and detection of amplicons from peanut DNA (nucleic acid to be detected) and competitor DNA (competitor nucleic acid)*

Polymerase chain reaction of peanut DNA in the presence or absence of competitor DNA was carried out in 0.2 ml thin-walled micro-tubes (Advanced Biotechnologies, Hamburg, Germany) at final volumes of 50 $\mu$l. The PCR mixture contained 1 x reaction buffer (20 mmol/l Tris HCl (pH 8.4), 50 mmol/l KCl; supplied with Platinum® *Taq,* Invitrogen, Karlsruhe, Germany), 5.0 mmol/l magnesium chloride (supplied with Platinum® *Taq,* Invitrogen); 200 $\mu$M each of the deoxyribonucleoside triphosphates (dNTPs) dATP, dCTP and dGTP, and 400 $\mu$M of dUTP as substitute for dTTP, and derived from a dUTP-containing dNTP mix (Carl Roth, Karlsruhe) where A is adenosine, C is cytidine, G is guanosine, U is uracil, and T is thymidine; 0.25 $\mu$g/$\mu$l of bovine serum albumin (Sigma, no. A 8022, Deisenhofen, Germany); 300 nmol/l of each primer 'forward' and 'reverse'; 200 nmol/l labeled peanut probe; 200 nmol/l labeled competitor probe; 0.5 units of uracil-N-glycosilase (AmpErase UNG, PE Biosystems, no. N 808-0096); 1.25 units of Platinum® *Taq* Gold polymerase (Invitrogen); and 1/10 of the reaction volume of undiluted extracted DNA (5 $\mu$l). The PCR was carried out in an Mx3005P™ QPCR system (Stratagene, via Agilent Technologies Sales & Services GmbH & Co. KG, Waldbronn, Germany) using the following conditions: chemical decontamination reaction from dUTP-containing template at 50 °C for 2 min, deactivation of UNG and activation of Platinum® *Taq* Gold polymerase at 95 °C for 2 min, followed by 45 two-step cycles with a DNA denaturation at 95 °C for 30 sec, and combined primer annealing and elongation at 62 °C for 30 sec. Fluorescence was recorded after each cycle at both the FAM and HEX channel.

*Preparation of chocolate spiked with peanut at defined levels*

Milk chocolate containing 100 ppm (0.01 %) peanut was prepared similar as described for hazelnut (Holzhauser T, Vieths S. Quantitative sandwich ELISA for determination of traces of hazelnut (Corylus avellana) protein in complex food matrixes. J Agric Food Chem 1999, 47:4209-4218): Peanut free chocolate (prechecked by peanut-specific in-house ELISA and real-time PCR) was spiked with finely ground peanut cream, that consisted of 100 % peanut (unknown variety), from a commercial retailer (GranoVita, #1008) at levels of 10 %, 1 %, 0.1 %, 0.01 %, and 0.001 %. 1 g of peanut was added to 9 g of a food sample, and the spiked sample was melted at 37 °C and mixed with a stirrer for better sample homogeneity. Thereafter, 1 g of the spiked sample containing 10 % of peanut was added to another 9 g of chocolate. The procedure was continued until a sample containing 0.001 % of peanut was obtained.

The accuracy of the spike levels in milk chocolate was verified with the in-house peanut specific ELISA in comparison to the peanut reference material 'peanut cream GranoVita' as was previously described for hazelnut (Holzhauser T, Vieths S. Quantitative sandwich ELISA for determination of traces of hazelnut (Corylus avellana) protein in complex food matrixes. J Agric Food Chem 1999, 47:4209-4218).

Each of the samples containing peanut at the concentration levels described was extracted according to the DNA extraction protocol for PCR analysis and according to protein extraction protocols for ELISA analysis.

*Samples with defined amount of peanut from official ring trials*

A milk chocolate with 2 ppm peanut, a dark chocolate with 20 ppm peanut, and a milk chocolate with 100 ppm peanut previously employed in a ring trial for performance testing as official German food regulatory method according to § 64 of the German "Lebensmittel-, Bedarfsgegenstande- und Futtermittelgesetzbuch" (LFGB) were included in this study. Each of the samples was extracted according to the DNA extraction protocol for PCR analysis and according to protein extraction protocols for ELISA analysis.

*Commercial food samples*

Commercial food samples were purchased from a local retailer.

*Calibration of real-time cqPCR for peanut quantification*

The competitor DNA was serially diluted to allow addition to the self prepared chocolate prior to DNA extraction. Identical portions of 300 mg chocolate, containing peanut at the 100 ppm level (exactly 75.9 ppm), were fortified with increasing amounts of competitor DNA. After DNA extraction, the FAM (peanut DNA probe) and HEX (competitor DNA probe) signals were recorded in real-time PCR allowing to determine the threshold cycles for the FAM and HEX signal at pre-defined fluorescence intensity. The amount of copy number of peanut DNA and competitor DNA was quantified against an external standard curve of peanut DNA PCR product and an external standard curve of competitor DNA, respectively. The logarithm of the copy ratio (peanut DNA/competitor DNA) was plotted against the logarithm of competitor DNA copies. After linear regression, the copy number necessary for equivalence of the copy numbers of peanut and competitor DNA was calculated from the linear equation.

*Semi-quantitative evaluation and quantitative determination by real-time cqPCR*

The real-time cq-PCR was calibrated to match 100 ppm (0.01 %) peanut in a chocolate sample. The semi-quantitative analysis allows to distinguish between the three cases A) peanut (FAM) and competitor (HEX) specific signal arise at identical threshold cycle ($C_T$): the sample contains 0.01 % peanut, B) $C_T$ (FAM) > $C_T$ (HEX): the sample contains more than 0.01 % peanut, and C) $C_T$ (FAM) < $C_T$ (HEX): the sample contains less than 0.01 % peanut. At the level of equivalence of both peanut and competitor specific signal, the detection is considered quantitative. Divergent signals allow semi-quantitative evaluation of the result.

Moreover, calculation of the amount of peanut in the sample can be done based on the following equation, provided that the efficiency of both the amplification of peanut DNA and competitor DNA is at or nearby 100 %:
Here:

$$\% \text{ peanut in sample} = 2^{\Delta Ct} \cdot (\% \text{ level of calibration})$$

$$= 2^{[Ct(\text{competitor}) - Ct(\text{peanut})]} \cdot (\% \text{ level of calibration});$$

$$\boxed{\% \text{ peanut in sample} = 0.01 \cdot 2^{Ct(\text{HEX}) - Ct(\text{FAM})}}$$

*In-house-ELISA for the quantification of peanut*

The sandwich-type ELISA for peanut quantification including protein extraction from food samples was performed as previously described (Stephan O, Vieths S. Development of a real-time PCR and a sandwich ELISA for detection of potentially allergenic trace amounts of peanut (Arachis hypogaea) in processed foods. J Agric Food Chem. 2004;52: 3754-60). The limit of detection is 1 ppm (mg/kg) peanut.

*Commercial ELISA for the quantification of peanut*

Three commercial peanut specific ELISAs, all certified as 'AOAC Research Institute Performance Tested Method', were included to compare to the quantitative peanut PCR in this study: BioKits Peanut Assay Kit (Tepnel BioSystems, via Coring Sytem Diagnostix GmbH, Gernsheim, Germany), RIDASCREEN®FAST Peanut (R-Biopharm AG, Darmstadt, Germany), and NEOGEN *Veratox*® Peanut Allergen Test (Neogen Europe Ltd., Auchincruive, Ayr, Scottland). All three commercial ELISAs feature peanut standards for direct read-out as 'peanut' without conversion from detectable peanut protein to peanut. The extraction buffers and protocols were included. The commercial ELISAs including sample preparation were performed according to the manufacturers' instructions.

**Results**

*Preparation of peanut spiked milk chocolate as a sample*

Peanut spikes were prepared as described in the materials section. The accuracy of the spikes was verified by the in-house peanut specific ELISA. This ELISA quantifies peanut protein. To determine the amount of peanut, quantified peanut protein is multiplied by a factor of conversion. For exact determination of peanut in a sample, the peanut to be quantified needs to be available as a reference. First, peanut protein from peanut cream 'Grano Vita' was quantified by the in-house peanut specific ELISA. The peanut cream had 29.15 % extractable and quanti-fiable peanut protein, resulting in a conversion factor of 3.43 to calculate the amount of peanut from quantified peanut protein in the spiked milk chocolate samples. The results of the quantified peanut in the spikes of milk chocolate are displayed in table 1.

*Optimization of the peanut specific PCR*

The amplification of an 86 bp peanut specific PCR product is based on the Ara h 2 gene (NCBI GenBank acc no. L77197). The method for amplification of peanut specific DNA was in principle desribed previously (Stephan O, Vieths S. Development of a real-time PCR and a sandwich ELISA for detection of potentially allergenic trace amounts of peanut (Arachis hypogaea) in processed foods. J Agric Food Chem. 2004;52: 3754-60). However, one primer mismatch was corrected and the PCR performance was optimized to obtain a PCR efficiency of almost 100 %. This was achieved by additionally changing the chemistry of the PCR reaction mixture including the hot start polymerase and the parameters of thermal cycling the PCR. The PCR was further performed as described herein.

*Generation of a competitor dsDNA*

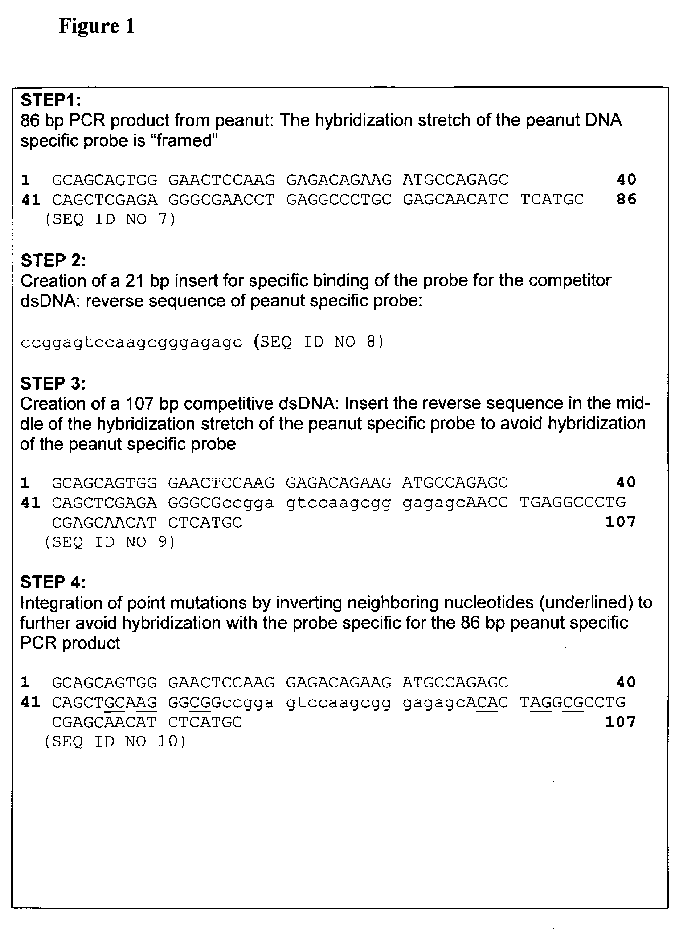

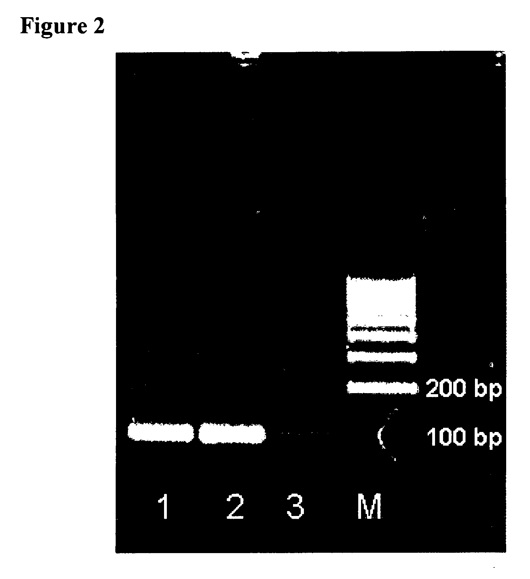

The 107 bp double-stranded competitor DNA was generated by the use of two overlapping synthetic oligonucleotides that were hybridized, the 3' ends filled up with Klenow fragment that lacks the 5' → 3' exonuclease activity of the native enzyme, and finally amplified in PCR with the peanut DNA specific primers and Taq Polymease. The underlying strategy to generate a competitive DNA with conserved primer binding sites but divergent probe hybridization stretches as well comparable composition of total nucleotides to allow differentiation of PCR products from peanut genomic DNA and competitor DNA by A) size for analysis in electrophoresis post PCR, and B) specific probes for real-time detection in PCR is given in figure 1. The quality of the purified competitor DNA PCR product was evaluated by agarose gel electrophoresis and fluorescence staining with ethidium bromide: By visual inspection, the competitor DNA PCR product comprised only one single DNA band of appropriate length (Figure 2). The serially diluted 107 bp competitor DNA was stored at -20 °C until use.

*Classic competitive PCR*

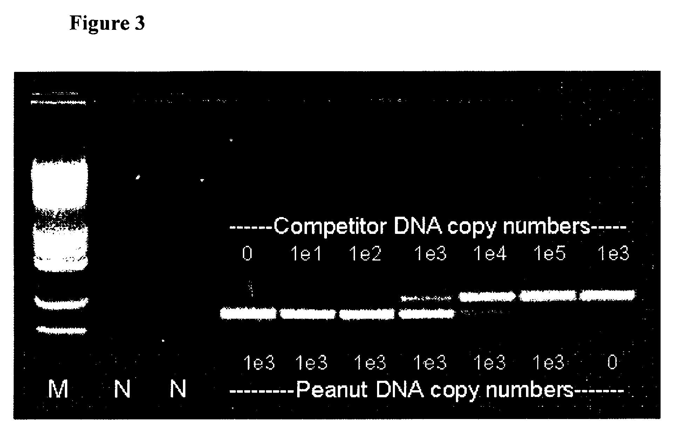

A classic competitive PCR with post-PCR agarose gel electrophoresis was run with a stock dilution of the 86 bp peanut DNA PCR product corresponding to approximately 1E3 copies, and increasing copies of 107 bp competitor for demonstrating the competitive reaction visually (figure 3). Obviously, PCR products of competitor and target (peanut) can be discriminated visually. However, determining the exact amount of competitor DNA to achieve identical amplification as for peanut DNA is difficult to achieve. Consequently, the competitive PCR was further set up for detection in real-time PCR.

*PCR efficiency and specificity for the peanut target DNA and competitor DNA*

Serial dilutions of peanut DNA PCR product were amplified in a non-competitive set-up, and a standard curve was recorded by plotting the $C_T$ of each standard versus the logarithm of the copy number of amplifiable DNA, both for peanut DNA and competitor DNA. The efficiency is 100 % at a slope of-3.322.

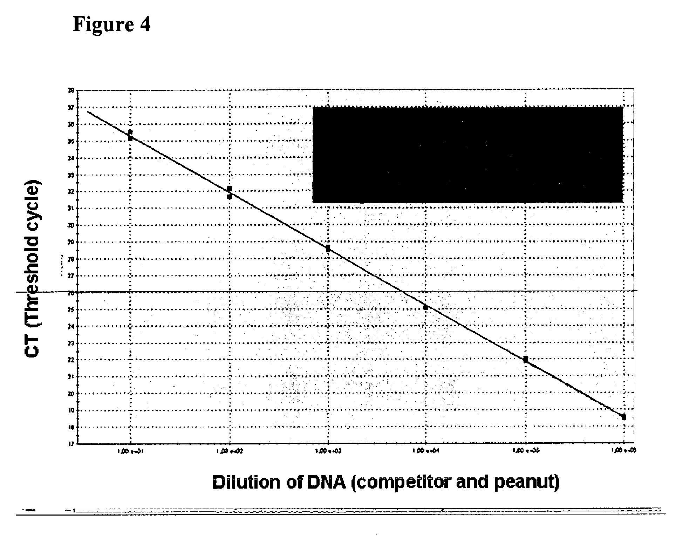

Figure 4 displays complete overlay of the standard curves originating from peanut DNA (filled squares) and competitor DNA (filled circles) with identical PCR efficiency of nearby 100 %.
Since both probes for peanut DNA and competitor DNA are present in the PCR reaction mix, fluorescence cross-talk of the sequence specific probes were measured for the FAM-labelled probe with competitor DNA, and for the HEX-labelled probe with peanut DNA. No fluorescence cross-talk between fluorescence labels and no false priming of the probes was observed.

*Calibration of competitive real-time PCR for a threshold of 100 ppm peanut*

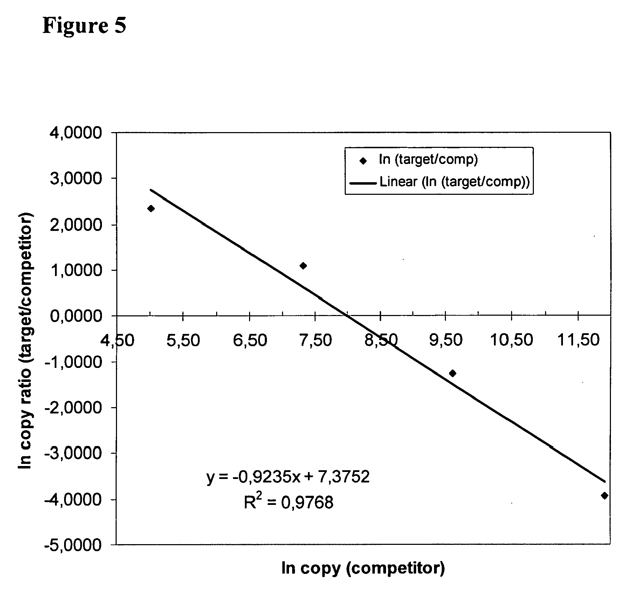

The milk chocolate spiked at the level of 100 ppm (0.01 %) peanut was used as reference for calibration of the competitive real-time PCR. As a first step, a rough titration with competitor DNA between 150 and 150,000 copies was done to identify the order of magnitude of competitor copies that are necessary for equal amplification in the presence of DNA originating from 100 ppm peanut. The calibration plot is given in figure 5. Resolution of the equation of the linear regression for 'y=0' resulted in 2940 copies of competitor. As the second step, a more narrow range of competitor DNA between 2000 and 4000 copies was chosen. The resolution of the equation of the linear regression resulted in 3112 copies of competitor DNA to compete equally with 100 ppm peanut. Since the true amount of peanut in the chocolate sample is 75.9 ppm, a total of 4100 copies are necessary to compete equally with exactly 100 ppm of peanut. For quantitative analysis of any unknown sample, 4100 copies of competitor DNA were added to each sample during the DNA extraction as described in the 'Materials' section.

*Semi-quantitative and quantitative determination of peanut in chocolate*

Each of the self-made peanut spiked chocolates were extracted in duplicate, each extract was quantified in two separate PCR reactions. The quantification was done based on the assumption of 100 % efficiency of the PCR for peanut and competitor DNA. The results are shown in table 2: 988 ppm and 75.9 ppm peanut were recovered at 122 and 113 %, respectively. The chocolate spiked with 6.86 ppm was slightly overestimated with a recovery of 187 %. The chocolates having 8944 and 96237 ppm were clearly overestimated, demonstrating that the quantitative range of the method spans approximately two orders of magnitude with each one order of magnitude above and below the level of calibration.

*Comparison of quantitative results of real-time cq-PCR and commercial ELISA for peanut in chocolate*

All investigations were performed in the year 2007 with the current versions of the commercial peanut specific ELISA. The ELISAs were performed according to the manufacturer's instructions: The quantification was each based on singlet determination of duplicate protein extractions. In PCR, each sample was extracted for DNA in duplicate, and each DNA extract was quantified in duplicate PCR reactions. The results are summarized in table 3.

Dark and milk chocolate spikes that ranged from 6.8 to 988 ppm peanut were quantified in PCR with recoveries between 89 % and 187 %. 2ppm were still detectable in 2/4 reactions with 81 % recovery. At the level of calibration, the recovery in PCR was 101 % and 113 %, respectively. By contrast, ELISA recoveries ranged between 5 % and 248%. Partially, peanut below 10 ppm was not detectable in ELISA, depending on the manufacturer. PCR also succeeded in quantifying peanut in matrix other than chocolate, such as nougat, without need for further calibration.

**Conclusions**

As a proof of concept, the normalized real-time PCR was at least as sensitive, specific and quantitative for peanut as were AOAC Research Institute Performance Tested peanut specific ELISA. Bearing in mind that peanut is the potential allergenic food for which most of the research and development of immunochemical methods has been put on, the potential power of the cqPCR method has been demonstrated. Moreover, several potentially allergenic sources may not be immunogenic enough to obtain high quality, high specificity antibodies to detect the allergenic food. By contrast, the cqPCR is independent from a successful immunization of animals and further can be designed according to oligonucleotide chemistry.

The advantage of this PCR invention over common antibody based techniques such as ELISA are (I) a completely reproducible chemistry including recombinant polymerases, (II) virtually infinite availability because of reproducible chemistry, (III) a very stable marker molecule because DNA is extraordinary heat stable in comparison to proteins, the latter of which are detected in ELISA, (IV) unparalleled specificity, (V) the avoidance of external calibration standard curves, (VI) internal calibration to normalize matrix effects, (VII) an internal calibrator (competitor) that also serves as inhibition control with regard to matrix effects, and finally a threshold based quantification that is efficient when verifying compliance with given analytical or regulatory thresholds.

In this example, samples were extracted in duplicate, and each sample DNA extract was analyzed in PCR in duplicate. In total four competitive PCR were run to obtain a quantitative result. For screening purposes to verify the presence of the targeted species, or to have a semi-quantitative result, one PCR run would suffice. Making use of modern real-time PCR thermo-cyclers with at least four fluorescence channels, this cqPCR further developed as a duplex analyte PCR can run in one reaction tube to quantify two different species simultaneously. Consequently, a screening of 8 different quantitative thresholds may be actualized with only 4 PCR reactions. In comparison to ELISAs that have a specific external calibration, usually with at least 4 standard points, cqPCR is also economically competitive.

This cqPCR can be calibrated against any available reference, provided it is representative for the whole species, including the presence of the species specific DNA.

In this example, calibration of the application to a certain threshold was done with external standard curves to quantify the ratio between copy numbers of peanut DNA and competitor DNA in the chocolate spike with 100 ppm peanut.

However, this can also be achieved empirically without knowing the absolute copy number of the competitor DNA but simply by applying a serial dilution of the stock of purified generated competitor DNA. Accordingly, the difference in threshold cycle ($\Delta C_T$) between competitor (HEX) and peanut (FAM) nucleic acid can be recorded at predefined fluorescence signal intensity. The $\Delta C_T$ is plotted against the logarithm of the factor of dilution of the serially diluted stock of the competitor DNA that has been generated as described. After linear regression of the semi-logarithmic data, the mathematical equation of the linear regression is solved for the factor of dilution of competitor DNA at $\Delta C_T$ equals zero. Finally, the competitor stock that is diluted according to the determined factor of dilution, serves as the internal standard to be added to any sample with unknown amount of organic substance to be quantified.

A quantification of the species of interest would be made possible on the basis of nucleic acid quantification techniques, however without quantifying the nucleic acids as an unnecessary interstate.

This format is especially powerful if integrated into a ready to use kit for the operator. This helps to reduce the steps of manipulation down to only a few steps up to full automation of the cqPCR including sample preparation.

**Tables**

Table 1: Quantified peanut in the spiked milk chocolate sample. (%CV, coefficient of variation of duplicate determination)

| spike level of peanut | | verified peanut spikes | |
| --- | --- | --- | --- |
| % | ppm (mg/kg) | ppm (mg/kg) | %CV |
| 10 | 100,000 | 96237 | 5.4 |
| 1 | 10,000 | 8944 | 5.9 |
| 0.1 | 1,000 | 988 | 10.0 |
| 0.01 | 100 | 75.9 | 1.8 |
| 0.001 | 10 | 6.86 | 0.5 |

**Table 2:** Quantification of peanut spiked into milk chocolate at a level between 10 to 100,000 ppm. Identification of the quantitative working range. (%CV, coefficient of variation; %R, recovery rate)

| Sample | Extraction no. | PCR no. | CT (HEX) | CT (FAM) | Δ CT (Hex-Fam) | % peanut | mean t | % peanut mean | % CV | % R |
|---|---|---|---|---|---|---|---|---|---|---|
| 0.001 % peanut (6.86 ppm) | 1 | 1 | 31.44 | 34.19 | -2.75 | 0.0014 | | | | |
| | 1 | 2 | 31.62 | 34.37 | -2.75 | 0.0014 | 0.0014 | | | |
| | 2 | 1 | 31.38 | 35.41 | -4.03 | 0.0006 | 0.0007 | **0.00128** | **35.3** | **187** |
| | 2 | 2 | 31.08 | 34.63 | -3.55 | 0.0008 | | | | |
| 0.01 % peanut (75.9 ppm) | 1 | 1 | 31.81 | 31.84 | -0.03 | 0.0098 | | | | |
| | 1 | 2 | 31.32 | 31.59 | -0.27 | 0.0082 | 0.0090 | | | |
| | 2 | 1 | 30.97 | 31.51 | -0.54 | 0.0067 | 0.0082 | **0.0086** | **4.7** | **113** |
| | 2 | 2 | 31.20 | 31.25 | -0.05 | 0.0096 | | | | |
| 0.1 % peanut (988 ppm) | 1 | 1 | 32.01 | 28.03 | 3.98 | 0.158 | | | | |
| | 1 | 2 | 31.71 | 28.14 | 3.57 | 0.119 | 0.138 | | | |
| | 2 | 1 | 31.62 | 28.34 | 3.28 | 0.097 | 0.103 | **0.121** | **14.7** | **122** |
| | 2 | 2 | 31.59 | 28.15 | 3.44 | 0.109 | | | | |
| 1 % peanut (8944 ppm) | 1 | 1 | 32.72 | 24.51 | 8.21 | 2.96 | | | | |
| | 1 | 2 | 33.37 | 24.69 | 8.68 | 4.10 | 3.53 | | | |
| | 2 | 1 | 32.37 | 24.22 | 8.15 | 2.84 | 3.04 | **3.29** | **7.5** | **368** |
| | 2 | 2 | 32.61 | 24.27 | 8.34 | 3.24 | | | | |
| 10 % peanut (96237 ppm) | 1 | 1 | 36.82 | 22.16 | 14.66 | 259 | | | | |
| | 1 | 2 | 37.76 | 22.08 | 15.68 | 525 | 392 | | | |
| | 2 | 1 | 39.01 | 22.78 | 16.23 | 769 | 1.169 | **781** | **49.8** | **>1000** |
| | 2 | 2 | 39.58 | 22.32 | 17.26 | 1.570 | | | | |

Table 3: Comparative quantification of peanut by ELISA and PCR. (choc., chocolate; % R, recovery rate; * 2/4 PCR positive).

| Sample type | matrix | ppm peanut known | cqPCR | | ELISA A | | ELISA B | | ELISA C | |
|---|---|---|---|---|---|---|---|---|---|---|
| | | | ppm peanut | % R | ppm peanut | % R | ppm peanut | % R | ppm peanut | % R |
| § 64 LFGB | milk choc. | 2 | 1.62* | 81 | 1.97 | 98 | 4.2 | 211 | < LOD | |
| § 64 LFGB | dark choc. | 20 | 17.9 | 89 | 7.2 | 36 | 13.6 | 68 | 1.1 | 5.4 |
| § 64 LFGB | milk choc. | 100 | 101 | 101 | 117 | 117 | 170.1 | 170 | 91.9 | 92 |
| own spike | milk choc. | 6.86 | 12.8 | 187 | 8.4 | 123 | 14.3 | 208 | 1.13 | 17 |
| own spike | milk choc. | 75.9 | 86.0 | 113 | 68.9 | 91 | 188.1 | 248 | 78.3 | 103 |

(continued)

| Sample type | matrix | ppm peanut known | cqPCR | | ELISA A | | ELISA B | | ELISA C | |
|---|---|---|---|---|---|---|---|---|---|---|
| | | | ppm peanut | % R | ppm peanut | % R | ppm peanut | % R | ppm peanut | % R |
| own spike | milk choc. | 988 | 1206 | 122 | 757 | 77 | 1884 | 191 | 908 | 92 |
| own spike | milk choc. | 0 | < LOD | | < LOD | | < LOD | | < LOD | |
| commercial | dark choc. | unknown | < LOD | | < LOD | | < LOD | | < LOD | |
| commercial | milk choc. | unknown | < LOD | | < LOD | | < LOD | | < LOD | |
| commercial | mousse | unknown | < LOD | | < LOD | | < LOD | | < LOD | |
| commercial | milk choc. | unknown | 11.4 | | 13.4 | | 11.5 | | 0.87 | |
| commercial | nougat choc. | unknown | 47.7 | | 47.4 | | 15.2 | | 38.5 | |

**Claims**

1. A method for the quantitative detection of an organic substance in a sample based on a given threshold value for that organic substance in the sample
through an amplification of a nucleic acid to be detected that stems from said organic substance,
comprising the steps of:

a) providing a given number of a second nucleic acid as a competitor (competitor nucleic acid) in an amplification of the nucleic acid to be detected, and adding the competitor nucleic acid to the sample, wherein the given number of the competitor nucleic acid results in an amplification in a signal that allows for deducing the amount of the organic substance in the sample relative to the given threshold value;

b) providing a first oligonucleotide and a second oligonucleotide for priming an amplification of the nucleic acid to be detected and of the competitor nucleic acid;

c) extracting the nucleic acid to be detected and the competitor nucleic acid from the sample;

d) providing

- a first detectable probe for hybridizing with the nucleic acid to be detected to generate a first detectable signal; and
- a second detectable probe for hybridizing with the competitor nucleic acid to generate a second detectable signal;

e) performing an amplification of the nucleic acid to be detected and simultaneously of the competitor nucleic acid; and

f) measuring the signal from the first detectable probe and the signal from the second detectable probe and deducing therefrom the amount of the organic substance in the sample relative to the given threshold value.

2. The method according to claim 1, wherein the amplification is performed through a real time polymerase chain reaction.

3. The method according to claim 2, wherein the deduction of the amount of the organic substance in the sample is performed by

- determining the $C_T$ value of the signal from the first detectable probe and of the signal from the second detectable probe representing the threshold value, and
- comparing the $C_T$ value from the first detectable probe with the $C_T$ value from the second detectable probe to deduce therefrom the amount of the organic substance in the sample relative to the threshold value.

4. The method according to claim 2 or 3, wherein the deduction of the amount of the organic substance in the sample comprises the following steps:

- if the $C_T$ value from the first detectable probe is substantially identical to the $C_T$ value from the second detectable probe, it is concluded that the amount of the organic substance in the sample is identical to the threshold value;
- if the $C_T$ value from the first detectable probe is below the $C_T$ value from the second detectable probe, it is concluded that the amount of the organic substance in the sample is higher than the threshold value.
- if the $C_T$ value from the first detectable probe is above the $C_T$ value from the second detectable probe, it is concluded that the amount of the organic substance in the sample is lower than the threshold value.

5. The method according to claim 3 or 4, wherein

- if PCR efficiency for both the organic substance and the competitor is identical and nearly 100 %, a difference in $C_T$ value from the first detectable probe to the $C_T$ value from the second detectable probe by 3.322 cycles results in a relative difference of tenfold compared to the calibrated amount.

6. The method according to claims 1 to 5, wherein the nucleic acid to be detected and the competitor nucleic acid both comprise

- a first sequence portion for hybridization with the first oligonucleotide, and
- a second sequence portion for hybridization with the second oligonucleotide.

7. The method according to claim 6, wherein

- the first sequence portion of the nucleic acid to be detected or of the competitor nucleic acid contains a sequence that is reverse complementary to the sequence of the first oligonucleotide, or
- the second sequence portion of the nucleic acid to be detected or of the competitor nucleic acid contains a sequence that is reverse complementary to the sequence of the second oligonucleotide.

8. The method according to claim 7, wherein the first sequence portion of the nucleic acid to be detected and the first sequence portion of the competitor nucleic acid are identical, or
wherein the second sequence portion of the nucleic acid to be detected and the second sequence portion of the competitor nucleic acid are identical.

9. The method according to claims 1 to 8, wherein the amplification product of the nucleic acid to be detected and of the competitor nucleic acid are identical in length.

10. The method according to claim 1 to 9, wherein the amplification product of the nucleic acid to be detected and the competitor nucleic acid differ in sequence in at least 1 nucleotide, preferably in 1 to 20 nucleotides.

11. The method according to claim 10, wherein the nucleic acid to be detected and the competitor nucleic acid differ in sequence only at a sequence portion where the detectable probe hybridizes.

12. The method according to claim 1 to 10, wherein determining the quantitative amount is performed by comparing the signal stemming from the first detectable probe with the signal stemming from the second detectable probe.

13. The method according to claim 1 to 12, wherein the first detectable probe and the second detectable probe are tagged with a detectable label, wherein the detectable label of the first detectable probe is distinguishable form the detectable label of the second detectable probe.

14. The method according to claim 1 to 13, wherein the quantitative detection is an absolute quantitative detection.

15. Kit for the quantitative detection of a nucleic acid in a sample, comprising

   a) a first oligonucleotide and a second oligonucleotide for priming an amplification of the nucleic acid to be detected
   b) a second nucleic acid as a competitor to a nucleic acid to be detected to which the first and the second oligonucleotide hybridize;
   c) a first detectable probe for hybridizing with the nucleic acid to be detected to generate a first signal, and a second detectable probe for hybridizing with the competitor nucleic acid to generate a second signal.

16. The kit according to claim 15, further comprising an enzyme for the amplification of the nucleic acid to be detected and for the amplification of the competitor nucleic acid.

17. The kit according to claim 15 or 16, further comprising a means for detecting the signal from the first detectable probe and from the second detectable probe.

## Figure 1

---

**STEP1:**
86 bp PCR product from peanut: The hybridization stretch of the peanut DNA specific probe is "framed"

```
 1  GCAGCAGTGG GAACTCCAAG GAGACAGAAG ATGCCAGAGC          40
41  CAGCTCGAGA GGGCGAACCT GAGGCCCTGC GAGCAACATC TCATGC    86
    (SEQ ID NO 7)
```

**STEP 2:**
Creation of a 21 bp insert for specific binding of the probe for the competitor dsDNA: reverse sequence of peanut specific probe:

```
ccggagtccaagcgggagagc (SEQ ID NO 8)
```

**STEP 3:**
Creation of a 107 bp competitive dsDNA: Insert the reverse sequence in the middle of the hybridization stretch of the peanut specific probe to avoid hybridization of the peanut specific probe

```
 1  GCAGCAGTGG GAACTCCAAG GAGACAGAAG ATGCCAGAGC          40
41  CAGCTCGAGA GGGCGccgga gtccaagcgg gagagcAACC TGAGGCCCTG
    CGAGCAACAT CTCATGC                                  107
    (SEQ ID NO 9)
```

**STEP 4:**
Integration of point mutations by inverting neighboring nucleotides (underlined) to further avoid hybridization with the probe specific for the 86 bp peanut specific PCR product

```
 1  GCAGCAGTGG GAACTCCAAG GAGACAGAAG ATGCCAGAGC          40
41  CAGCTGCAAG GGCGGccgga gtccaagcgg gagagcACAC TAGGCGCCTG
    CGAGCAACAT CTCATGC                                  107
    (SEQ ID NO 10)
```

**Figure 2**

**Figure 3**

## Figure 4

**Dilution of DNA (competitor and peanut)**

# Figure 5

In copy ratio (target/competitor) vs In copy (competitor)

Legend:
- ♦ In (target/comp)
- —— Linear (In (target/comp))

$y = -0,9235x + 7,3752$
$R^2 = 0,9768$

# Figure 6

$y = -1,5795x + 12,704$

$R^2 = 0,9946$

ln copy (competitor)

Europäisches
Patentamt

European
Patent Office

Office européen
des brevets

# EUROPEAN SEARCH REPORT

Application Number

EP 08 02 0918

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GIBSON U E M ET AL: "A NOVEL METHOD FOR REAL TIME QUANTITATIVE RT-PCR" GENOME RESEARCH, COLD SPRING HARBOR LABORATORY PRESS, WOODBURY, NY, US, vol. 6, no. 10, 1 October 1996 (1996-10-01), pages 995-1001, XP000642796 ISSN: 1088-9051 | 1-4, 6-10, 12-14 | INV. C12Q1/68 |
| Y | * the whole document * | 5,11,16, 17 | |
| X | DE 199 06 169 A1 (BIOINSIDE GES FUER BIODIAGNOST [DE]) 10 August 2000 (2000-08-10) | 1-4, 6-10, 12-15 | |
| Y | * the whole document * | 5,11,16, 17 | |
| X | SECCHIERO P ET AL: "QUANTITATIVE PCR FOR HUMAN HERPESVIRUSES 6 AND 7" JOURNAL OF CLINICAL MICROBIOLOGY, WASHINGTON, DC, US, vol. 33, no. 8, 1 August 1995 (1995-08-01), pages 2124-2130, XP000564243 ISSN: 0095-1137 | 1-4, 6-10, 12-14 | TECHNICAL FIELDS SEARCHED (IPC) C12Q |
| Y | * the whole document * | 5,11,16, 17 | |

-/--

The present search report has been drawn up for all claims

| Place of search | Date of completion of the search | Examiner |
|---|---|---|
| The Hague | 21 July 2009 | Franz, Cerstin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches
Patentamt

European
Patent Office

Office européen
des brevets

**EUROPEAN SEARCH REPORT**

Application Number

EP 08 02 0918

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,Y | STEPHAN OLIVER ET AL: "Development of a real-time PCR and a sandwich ELISA for detection of potentially allergenic trace amounts of peanut (Arachis hypogaea) in processed foods." JOURNAL OF AGRICULTURAL AND FOOD CHEMISTRY 16 JUN 2004, vol. 52, no. 12, 16 June 2004 (2004-06-16), pages 3754-3760, XP002538155 ISSN: 0021-8561 * the whole document * | 1-17 | |
| Y | HIRD H ET AL: "Detection of peanut using real-time polymerase chain reaction" EUROPEAN FOOD RESEARCH AND TECHNOLOGY ; ZEITSCHRIFT FÜR LEBENSMITTELUNTERSUCHUNG UND -FORSCHUNG A, SPRINGER, BERLIN, DE, vol. 217, no. 3, 1 September 2003 (2003-09-01), pages 265-268, XP019327991 ISSN: 1438-2385 * the whole document * | 1-17 | |
| A | HEID C A ET AL: "REAL TIME QUANTITATIVE PCR" GENOME RESEARCH, COLD SPRING HARBOR LABORATORY PRESS, WOODBURY, NY, US, vol. 6, no. 10, 1 October 1996 (1996-10-01), pages 986-994, XP000642795 ISSN: 1088-9051 * the whole document * | 1-17 | |

TECHNICAL FIELDS
SEARCHED (IPC)

-/--

The present search report has been drawn up for all claims

| Place of search | Date of completion of the search | Examiner |
|---|---|---|
| The Hague | 21 July 2009 | Franz, Cerstin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 08 02 0918

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HUBNER P ET AL: "Validation of PCR methods for quantitation of genetically modified plants in food" JOURNAL OF AOAC INTERNATIONAL, AOAC INTERNATIONAL, ARLINGTON, VA, US, vol. 84, no. 6, 1 November 2001 (2001-11-01), pages 1855-1864, XP002981728 ISSN: 1060-3271 * the whole document * | 1-17 | |
| A | STUDER E ET AL: "QUANTITATIVE COMPETITIVE PCR FOR THE DETECTION OF GENETICALLY MODIFIED SOYBEAN AND MAIZE" ZEITSCHRIFT FUER LEBENSMITTEL-UNTERSUCHUNG UND -FORSCHUNG. A,EUROPEAN FOOD RESEARCH AND TECHNOLOGY, SPRINGER, HEIDELBERG, DE, vol. 207, no. 3, 1 January 1998 (1998-01-01), pages 207-213, XP000937499 ISSN: 1431-4630 * the whole document * | 1-17 | |

TECHNICAL FIELDS
SEARCHED (IPC)

The present search report has been drawn up for all claims

| Place of search | Date of completion of the search | Examiner |
|---|---|---|
| The Hague | 21 July 2009 | Franz, Cerstin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 08 02 0918

This annex lists the patent family members relating to the patent documents cited in  the above-mentioned European search report.
The members are as contained in the European Patent Office EDP file on
The European Patent Office is in no way liable for these particulars which are merely given for the purpose of information.

21-07-2009

| Patent document cited in search report | | Publication date | Patent family member(s) | | Publication date |
|---|---|---|---|---|---|
| DE 19906169 | A1 | 10-08-2000 | AT | 239802 T | 15-05-2003 |
| | | | WO | 0047764 A2 | 17-08-2000 |
| | | | EP | 1144676 A2 | 17-10-2001 |
| | | | JP | 2002536024 T | 29-10-2002 |
| | | | US | 2003148278 A1 | 07-08-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Poms et al.** *Food additives and contaminants,* 2005, vol. 22, 104-112
- **Holzhauser et al.** *Eur Food Res Technol,* 2000, vol. 211, 360-365
- **Hird et al.** *Eur Food Res Technol,* 2003, vol. 217, 265-268
- **Stephan ; Vieths.** *J Agric Food Chem,* 2004, vol. 52, 3754-3760

- **Holzhauser T ; Vieths S.** Quantitative sandwich ELISA for determination of traces of hazelnut (Corylus avellana) protein in complex food matrixes. *J Agric Food Chem,* 1999, vol. 47, 4209-4218
- **Stephan O ; Vieths S.** Development of a real-time PCR and a sandwich ELISA for detection of potentially allergenic trace amounts of peanut (Arachis hypogaea) in processed foods. *J Agric Food Chem.,* 2004, vol. 52, 3754-60

SEQUENCE LISTING

<110> Paul-Ehrlich-Institut Bundesamt für Sera und Impfstoffe

<120> Method for the Quantitative Detection of an Organic Substance in a Sample

<130> FBP20957

<150> EP 08015740.7
<151> 2008-09-05

<160> 10

<170> PatentIn version 3.3

<210> 1
<211> 26
<212> DNA
<213> Artificial

<220>
<223> Forward PCR primer

<400> 1
gcagcagtgg gaactccaag gagaca                                        26

<210> 2
<211> 20
<212> DNA
<213> Artificial

<220>
<223> Reverse PCR primer

<400> 2
gcatgagatg ttgctcgcag                                               20

<210> 3
<211> 21
<212> DNA
<213> Artificial

<220>
<223> Peanut DNA specific probe

<220>
<221> 5'FAM
<222> (1)..(1)

<220>
<221> 3'BHQ
<222> (21)..(21)

```
<400>  3
cgagagggcg aacctgaggc c                                         21


<210>  4
<211>  21
<212>  DNA
<213>  Artificial

<220>
<223>  Competitor DNA specific probe


<220>
<221>  5'HEX
<222>  (1)..(1)

<220>
<221>  3'BHQ
<222>  (21)..(21)


<400>  4
ccggagtcca agcgggagag c                                         21


<210>  5
<211>  76
<212>  DNA
<213>  Artificial

<220>
<223>  Synthesis oligo #1

<400>  5
gcagcagtgg gaactccaag gagacagaag atgccagagc cagctgcaag ggcggccgga   60

gtccaagcgg gagagc                                               76


<210>  6
<211>  52
<212>  DNA
<213>  Artificial

<220>
<223>  Synthesis oligo #2

<400>  6
gcatgagatg ttgctcgcag gcgcctagtg tgctctcccg cttggactcc gg         52


<210>  7
<211>  86
<212>  DNA
<213>  Artificial
```

```
<220>
<223>  Probe

<400>  7
gcagcagtgg gaactccaag gagacagaag atgccagagc cagctcgaga gggcgaacct     60

gaggccctgc gagcaacatc tcatgc                                          86


<210>  8
<211>  21
<212>  DNA
<213>  Artificial

<220>
<223>  Primer

<400>  8
ccggagtcca agcgggagag c                                               21


<210>  9
<211>  107
<212>  DNA
<213>  Artificial

<220>
<223>  Probe

<400>  9
gcagcagtgg gaactccaag gagacagaag atgccagagc cagctcgaga gggcgccgga     60

gtccaagcgg gagagcaacc tgaggccctg cgagcaacat ctcatgc                  107


<210>  10
<211>  107
<212>  DNA
<213>  Artificial

<220>
<223>  Probe

<400>  10
gcagcagtgg gaactccaag gagacagaag atgccagagc cagctgcaag ggcggccgga     60

gtccaagcgg gagagcacac taggcgcctg cgagcaacat ctcatgc                  107
```